Europäisches Patentamt

European Patent Office

Office européen des brevets

(11) Veröffentlichungsnummer: **0 226 918**

**A1**

# (12) EUROPÄISCHE PATENTANMELDUNG

(21) Anmeldenummer: 86116989.4

(22) Anmeldetag: 06.12.86

(51) Int. Cl.⁴: **C07D 307/89**

(30) Priorität: 12.12.85 DE 3543822

(43) Veröffentlichungstag der Anmeldung:
01.07.87 Patentblatt 87/27

(84) Benannte Vertragsstaaten:
**BE DE FR GB IT NL SE**

(71) Anmelder: **BASF Aktiengesellschaft**
**Carl-Bosch-Strasse 38**
**D-6700 Ludwigshafen(DE)**

(72) Erfinder: **Eichorn, Hans-Dieter, Dr.**
**Buchnerstrasse 13**
**D-6700 Ludwigshafen(DE)**
Erfinder: **Menger, Volkmar, Dr.**
**Pfalzgrafenstrasse 33**
**D-6730 Neustadt(DE)**
Erfinder: **Reuter, Peter, Dr.**
**Holbeinstrasse 19**
**D-6800 Mannheim 25(DE)**
Erfinder: **Schmidt,Johannes E.,Dr.**
**Pariser Strasse 23**
**D-6700 Ludwigshafen(DE)**
Erfinder: **Schwarzmann, Matthias, Dr.**
**Carl-Bosch-Strasse 54**
**D-6703 Limburgerhof(DE)**

(54) **Verfahren zur katalytischen Oxidation von 4-tertiär-Butyl-o-Xylol.**

(57) Die Anmeldung betrifft ein Verfahren zur Herstellung von 4-tertiär-Butyl-Phthalsäureanhydrid durch Oxidation von 4-tertiär-Butyl-o-Xylol mit Luft, Sauerstoff oder Sauerstoff enthaltenden Gasen in Gegenwart von Katalysatoren, wobei ein geformter Katalysator verwendet wird, der als aktive Komponenten Titandioxid, überwiegend in der Anatasmodifikation und Vanadiumpentoxid enthält.

Der Katalysator kann zusätzlich als Promotoren Antimon, Niob, Alkalimetalle, Phosphor und Zirkon oder Verbindungen dieser Elemente enthalten und auf einem inerten Träger aus Magnesiumsilikat, vorzugsweise in Ringform oder Schalenform, aufgetragen sein.

## Verfahren zur katalytischen Oxidation von 4-tertiär-Butyl-o-Xylol

Die Erfindung betrifft ein Verfahren zur Herstellung von 4-tertiär-Butyl-Phthalsäureanhydrid durch katalytische Oxidation von 4-tertiär-Butyl-o-Xylol mit Luft, Sauerstoff oder Sauerstoff enthaltenden Gasen an Vanadiumpentoxid und Titandioxid enthaltenden geformten Katalysatoren.

Zur Oxidation von 4-tertiär-Butyl-o-Xylol zu 4-tertiär-Butyl-Phthalsäureanhydrid sind bereits verschiedene Verfahren vorgeschlagen worden. So wird in der JP-OS-7 352 733 vorgeschlagen, 0,283 Vol.-% 4-tertiär-Butyl-o-Xylol in Luft bei 410°C über einen Katalysator zu leiten, der hergestellt wird, indem 20 Teile Vanadiumpentoxid in 60 Teilen konzentrierter Salzsäure aufgelöst, mit 200 Teilen Wasser vermischt und dazu 200 Teile Aluminiumoxid zugegeben wird. Danach soll die Mischung getrocknet und 3 Stunden bei 500°C calciniert werden.

In der SU-385 947 wird vorgeschlagen, 4-tertiär-Butyl-o-Xylol an einem mit $WO_3$, $Al_2O_3$, $P_2O_5$ und $Ce_2O_3$ promotierten Vanadiumpentoxid enthaltenden Katalysator mit Luft zu 4-tertiär-Butyl-Phthalsäureanhydrid umzusetzen.

Bei der Durchführung dieser genannten Verfahren und unter Verwendung dieser Katalysatoren wird allerdings festgestellt, daß insbesondere bei höheren Konzentrationen von 4-tertiär-Butyl-o-Xylol im Einsatzgas, bei höheren Durchsätzen sowie bei hohen Produktionsleistungen nur unbefriedigende Ergebnisse erzielt werden. Der Grund liegt darin, daß bei diesen Verfahren unter Verwendung der genannten Katalysatoren sehr hohe Druckverluste auftreten, die die durchzusetzende Gasmenge sehr begrenzen. Da die Oxidation von 4-tertiär-Butyl-o-Xylol außerdem stark exotherm ist, treten insbesondere bei höheren Konzentrationen von 4-tertiär-Butyl-o-Xylol im Einsatzgas, bei höheren Durchsätzen sowie bei höheren Produktionsleistungen sogenannte heiße Stellen (hot spots) in der Katalysatorschicht auf, die eine übermäßige Oxidationsreaktion induzieren, womit durch die Bildung von Kohlenmonoxid und Kohlendioxid ein markanter Abfall der Ausbeute an Wertprodukten wie z.B. 4-tertiär-Butyl-Phthalsäureanhydrid erfolgt. Außerdem führen solche hot spots zu einer Schädigung des Katalysators, die die Wirtschaftlichkeit eines solchen Verfahrens sehr begrenzt.

Aus diesem Grund wurden auch Verfahren vorgeschlagen, bei denen die Oxidation dieser Verbindung in flüssiger Phase durchgeführt wird. So können nach der SU-PS-495 905 Alkyl-o-Xylole durch Oxidation mit Sauerstoff in Gegenwart von Cobaltacetat und Natriumbromid in Essigsäure als Reaktionsmedium zu den entsprechenden Alkylphthalsäuren umgesetzt werden.

Weiterhin kann nach J. Chem. Soc. (London), 1949, Seite 1314, 4-tertiär-Butyl-o-Xylol mit Kaliumpermanganat zu 4-tertiär-Butyl-Phthalsäure oxidiert und anschließend mit Acetanhydrid zu 4-tertiär-Butyl-Phthalsäureanhydrid umgesetzt werden.

Flüssigphasenverfahren können aber nur unter hohem technischen Aufwand kontinuierlich betrieben werden. Außerdem können bei diesen Verfahren oft Probleme, z.B. bei der Katalysatoraufarbeitung oder bei der Produktaufarbeitung auftreten. Beispielsweise werden bei der Oxidation von 4-tertiär-Butyl-o-Xylol mit Salpetersäure Nitroverbindungen gebildet, die zum einen die Ausbeute herabsetzen und zum anderen die Produktaufarbeitung erheblich erschweren, da solche Nitroverbindungen thermisch bzw. mechanisch instabil sind und zur spontanen Zersetzung neigen. Deshalb sind solche Verfahren nur selten sicher und einfach zu beherrschen, zumal dann, wenn die Verfahren mit großen Durchsätzen betrieben werden sollen.

Der vorliegenden Erfindung liegt damit die Aufgabe zugrunde, ein Verfahren zur Herstellung von 4-tertiär-Butyl-Phthalsäureanhydrid durch partielle Oxidation von 4-tertiär-Butyl-o-Xylol mit Luft, Sauerstoff oder Sauerstoff enthaltenden Gasen in Gegenwart von Katalysatoren zu entwickeln, welches über einen langen Zeitraum mit hohen Produktausbeuten, großen Durchsätzen, großen Produktionsleistungen und damit technisch einfach und sicher betrieben werden kann.

Die Erfindung besteht darin, daß man die Oxidation in Gegenwart von geformten Katalysatoren, die als aktive Komponenten Titandioxid, überwiegend in der Anatasmodifikation und Vanadinpentoxid enthalten.

Vorteilhaft werden Katalysatoren verwendet, die als Promotoren zusätzlich Antimon, Niob, Alkalimetalle, Phosphor und Zirkon oder Verbindungen dieser Elemente enthalten. Zweckmäßig werden sogenannte Vollkatalysatoren verwendet. Man kann aber auch Katalysatoren verwenden, die die Aktivmasse auf einem inerten Träger aus Magnesiumsilikat, vorzugsweise in Ringform, oder Schalenform aufgetragen enthalten.

Es war überraschend, daß man auf dem Wege der katalytischen Luftoxidation 4-tertiär-Butyl-Phthalsäureanhydrid aus 4-tertiär-Butyl-o-Xylol herstellen kann.

Nach DE-PS-2 250 448 oder DE-PS-2 118 871 erhält man bei der Luftoxidation von 1,2,4-Trimethylbenzhol eine Oxidation aller drei Methylgruppen zur Carboxylgruppe. Andererseits geht aus "Houben-Weyl = Methoden der organischen Chemie", 1952, Band 8, Seite 385 ff hervor, daß alle aliphatischen Seitenketten am Aromaten sehr leicht oxidiert werden können. Dabei werden auch längere und auch verzweigte Ketten meist vollständig zur kernständigen Carboxylgruppe oxidiert, wobei längere Ketten in der Regel zuerst abgebaut werden.

Bei der Durchführung des erfindungsgemäßen neuen Verfahrens können die Konzentrationen von 4-tertiär-Butyl-o-Xylol im Sauerstoff enthaltenden Gas in weiten Grenzen gewählt werden. Zum Beispiel kann im Falle von Luft als Sauerstoff enthaltendes Gas die Beladung von 4-tertiär-Butyl-o-Xylol im Bereich zwischen 10 und 120 g/Nm³ Luft, vorzugsweise zwischen 20 und 90 g/Nm³ zugeführter Luft liegen.

Der lichte Durchmesser der Reaktorrohre richtet sich unter anderem nach den Abmessungen der Feststoffschüttung, bestehend aus dem verwendeten, geformten Katalysator sowie dem gegebenenfalls verwendeten geformten Inertmaterial und liegt zwischen 15 mm und 40 mm, vorzugsweise zwischen 19 mm und 30 mm. Die Länge eines Reaktorrohres kann ebenfalls in weiten Grenzen variiert werden und liegt im allgemeinen zwischen 1 und 10 m. Die Reaktorrohre selbst sind üblicherweise zu einem Rohrbündel angeordnet, wobei sich die Anzahl der Rohre nach der erforderlichen Produktionsleistung richtet. Zur Beheizung bzw. zur Temperierung der Rohre können die üblichen, verschiedenen Wärmeübertragungsmedien verwendet werden. Bevorzugt werden Salzschmelzen, die je nach den erforderlichen Temperaturen unterschiedliche Zusammensetzungen aufweisen können, und welche dem Fachmann hinreichend bekannt sind. Die Temperatur des Wärmeübertragungsmediums richtet sich nach den Durchsätzen, der Konzentration von 4-tertiär-Butyl-o-Xylol im Sauerstoff enthaltenden Gas, den erforderlichen Umsätzen sowie der Aktivität und der Selektivität der verwendeten Katalysatoren und gegebenenfalls noch nach der Form der verwendeten Katalysatoren und des verwendeten Inertmaterials und liegt im allgemeinen zwischen 200°C und 500°C. Bei zu niedrigen Temperaturen wird die Reaktion nicht anspringen, während bei zu hohen Temperaturen eine übermäßige, unerwünschte Reaktion von 4-tertiär-Butyl-o-Xylol zu CO und $CO_2$ erfolgen wird.

Von Fall zu Fall kann es sich auch als günstig erweisen, den Reaktor in mehrere Stufen, z.B. in zwei, drei oder mehrere einzelne Reaktoren zu unterteilen, die bei Bedarf getrennt temperiert und somit auch auf unterschiedlichem Temperaturniveau gehalten werden können. Bei einer solchen Anordnung der Reaktoren können auch einzelne Einsatzstoffe, z.B. Sauerstoff, Luft, ein sauerstoffenthaltendes Gas oder 4-tertiär-Butyl-o-Xylol in verschiedenen Verhältnissen unterteilt und einzelnen Reaktoren getrennt zugeführt werden. Eine solche Vorgehensweise bietet sich beispielsweise dann an, wenn das Verfahren bei höheren Durchsätzen oder z.B. bei höheren Konzentrationen an 4-tertiär-Butyl-o-Xylol oder Sauerstoff im Einsatzgas betrieben werden soll.

Der Druck, bei dem das neue Verfahren betrieben wird, ist nicht kritisch und liegt im allgemeinen in einem Bereich von 1 bis 50 bar, vorzugsweise bei Drücken bis zu 10 bar. Die Raumgeschwindigkeit (Volumen der zugeführten Gasmenge pro Stunde geteilt durch das Katalysatorschüttvolumen) hängt von der Konzentration von 4-tertiär-Butyl-o-Xylol im sauerstoffenthaltenden Gas, den erforderlichen Umsätzen, der Aktivität und Selektivität des Katalysators ab und liegt in einem Bereich von 100 bis 20 000 vorzugsweise von 1000 bis 10 000.

In einer Ausführungsform des neuen Verfahrens werden die den letzten Reaktor verlassenden Gase einem geeigneten Produktabscheidesystem zugeführt und das von den Wertkomponenten befreite Restgas auf eine geeignete Weise entsorgt. Bei einer weiteren Ausführungsform des neuen Verfahrens werden die den Reaktor verlassenden Gase gänzlich oder auch teilweise von den Wertprodukten wie 4-tertiär-Butyl-Phthalsäureanhydrid befreit und das Restgas, gegebenenfalls nicht umgesetztes 4-tertiär-Butyl-o-Xylol enthaltend, dem Reaktor bzw. bei mehrstufiger Fahrweise einem der Reaktoren, vorzugsweise dem ersten Reaktor wieder zugeführt. Gegebenenfalls kann das Restgas auch unterteilt und verschieden Reaktoren wieder zugeführt werden.

Erfindungsgemäß wird ein Vanadiumpentoxid und Titandioxid, gegebenenfalls noch Promotoren enthaltender, geformter Katalysator verwendet. Dieser Katalysator kann sowohl vollständig aus der aktiven Masse bestehen (Vollkatalysator) oder auch die aktive Masse auf einem inerten, stabilen Träger aufgebracht, enthalten.

Die aktive Masse der Katalysatoren besteht aus 1 bis 30 Gew.-%, vorzugsweise 2 bis 10 Gew.-% an Vanadiumpentoxid und 70 bis 99 Gew.-%, vorzugsweise 90 bis 98 Gew.-%, an Titandioxid, das überwiegend in der Modifikation von Anatas vorliegt. Das verwendete Titandioxid weist eine spezifische Oberfläche nach BET von 7 bis 100 m²/g, vorzugsweise von 9 bis 60 m²/g auf. Weiter-

hin kann das verwendete Titandioxid Phosphor enthalten, und zwar in einem Anteil von 0,01 bis 0,7 Gew.-% Phosphor, bezogen auf die Gesamtmasse von Titandioxid.

Zur Erhöhung der Aktivität, der Selektivität oder der Standzeit der Katalysatoren können der Aktivmasse noch Promotoren zugefügt werden. Solche Promotoren können Verbindungen des Antimons, des Niobs, Alkalimetallverbindungen, Phosphorverbindungen oder Zirkonverbindungen sein.

Der Anteil der Promotoren in der Katalysatormasse ist wechselnd und richtet sich nach der gewünschten Aktivität bzw. Selektivität des Katalysators.

Im allgemeinen beträgt der Anteil an Niob bzw. Antimon in der Katalysatoraktivmasse zwischen 0,5 bis 10 Gew.-%, vorzugsweise zwischen 1,5 bis 4 Gew.-% an $Nb_2O_5$ bzw. $Sb_2O_3$, bezogen auf die gesamte Katalysatoraktivmasse. Diese Oxide können bereits als solche eingesetzt werden. Es können jedoch auch solche Verbindungen dieser Elemente verwendet werden, wie z.B. die Chloride, die durch Temperaturbehandlung in die entsprechenden Oxide überführt werden. Bei Verwendung von Zirkonoxid als Promotor kann der Anteil dieser Verbindung bis zu 30 Gew.-%, bezogen auf das eingesetzte Titandioxid betragen.

Der Anteil der Alkalimetallverbindung, vorzugsweise eine oder mehrere Verbindungen von Natrium, Kalium, Rubidium und/oder Caesium liegt zwischen 0,01 und 1,0 Gew.-%, vorzugsweise zwischen 0,05 und 0,6 Gew.-% Alkalimetall, bezogen auf die gesamte Katalysatoraktivmasse. Vorzugsweise werden als Alkalimetallverbindungen solche Verbindungen eingesetzt, die bei Temperaturbehandlung in die Oxide übergehen. Solche Verbindungen sind z.B. die Nitrate, die Carbonate, die Hydrogencarbonate, die Sulfate, die Hydroxide oder Alkalisalze organischer Säuren wie z.B. die Tartrate, die Acetate, usw., oder andere organische Alkalimetallverbindungen wie z.B. die Acetylacetonate usw.

Außer dem im eingesetzten Titandioxid bereits enthaltenen Phosphor kann der Katalysatoraktivmasse zur Aktivierung zusätzlich Phosphor zugesetzt werden. Bevorzugt werden Phosphorverbindungen zugesetzt wie zum Beispiel Phosphorsäuren oder Phosphate wie z.B. $(NH_4)_2HPO_4$ oder $NH_4H_2PO_4$ usw. Der Anteil an zugesetztem Phosphor kann bis zu 1,5 Gew.-%, bevorzugt bis 0,8 Gew.-%, an Phosphor betragen.

Die Herstellung des Katalysators kann auf verschiedene Art und Weise erfolgen:

Ein Herstellungsweg geht aus von feinverteiltem Titandioxid, überwiegend in der Anatasmodifikation vorliegend, auf welches man eine Lösung oder eine Suspension einer Vanadiumverbindung aufbringt. Als Vanadiumverbindungen kommen Vanadiumpentoxid oder solche Vanadiumverbindungen in Betracht, die bei höherer Temperatur in Vanadiumpentoxid übergehen, z.B. Vanadyloxalat, Vanadylformiat, Vanadyltartrat oder Ammoniumvanadat. Auch Mischfällungen von Titandioxid oder Titan-Vanadiumverbindungen, z.B. Titanvanadate, können eingesetzt werden. Das auf den Träger aufzubringende Gemisch kann z.B. so hergestellt werden, daß man das feinverteilte Titandioxid mit der Lösung oder der Suspension der Vanadiumverbindung in Wasser und/oder einem organischen Lösungsmittel wie Formamid oder Ethanolamin mischt oder die Lösung bzw. die Suspension auf das Titandioxid aufsprüht.

Der Zusatz der Promotoren zur Aktivmasse kann gleichzeitig mit der Tränkung des Titandioxids mit der Vanadiumverbindung erfolgen, indem die entsprechende Verbindung des Promotors der Imprägnierlösung bzw. der Suspension beigefügt wird. Andererseits können jedoch die Promotoren auch durch mehrere Tränkschritte entweder vor oder nach dem Aufbringen der Vanadiumverbindung imprägniert werden. Zirkondioxid, Nioboxid oder Antimonoxid können auch bereits vor dem ersten Imprägnierschritt mit dem Titandioxid auf eine geeignete Weise vermischt werden.

Nach dem letzten Imprägnierschritt, gegebenenfalls auch nach jeder einzelnen Tränkung, erfolgt ein mehrstündiges Trocknen der imprägnierten Masse, in der Regel im Bereich von Raumtemperatur bis etwa 350°C, entweder in Luft oder in Inertgasatmosphäre, wie z.B. Stickstoff, wobei auch eine stufenweise Trocknung von Vorteil sein kann.

Die getrocknete Aktivmasse wird anschließend zum geformten Katalysator weiterverarbeitet. Zur Formgebung können bekannte Verfahren wie zum Beispiel die Tablettierung, die Extrudierung oder die Granulierung herangezogen werden. Bei der Formgebung durch Tablettierung können der Aktivmasse ein oder mehrere Zusätze, wie z.B. Bindemittel und/oder Schmiermittel (Gleitmittel) zugegeben werden. Als Beispiele für solche Zusätze seien Graphit, Stearinsäure und deren Metallsalze, Talkum, Methylcellulose bzw. andere modifizierte Cellulosen, Glycerinmonostearat, Polyethylenglykole, Polyvinylalkohole, Polyvinylpyrrolidone, Polyacrylester u.ä. genannt. Bevorzugt durch Tablettierung hergestellte Formkörper sind Tabletten oder rohrförmige Formkörper wie Ringe.

Bei der Formgebung durch Extrudieren wird die Aktivmasse in der Regel mit Wasser sowie unter Zusatz von Extrudierhilfsmitteln wie Stärke, Methylcellulose, Graphit, Polyethylenglykole, Polyvinylalkohole, Polyvinylpyrrolidone, Polyacrylester, Stearinsäure und deren Metallsalze, Naphthalin, Ammoniak, Ameisensäure, Oxalsäure, Salpetersäure etc. entweder als einem porositätsverbessernden Mittel oder als Gleitmittel oder als Peptisierungsmittel verformt oder zunächst geknetet und anschließend verformt; bevorzugt ist dabei die Knetung und die anschließende Verformung, insbesondere das Extrudieren. Die bei der Extrusion erhaltenen Formlinge werden zunächst bei Temperaturen im Bereich von Raumtemperatur bis 200°C getrocknet und danach bei Temperaturen bis zu 500°C calciniert, wobei auch eine stufenweise Vorcalcination von Vorteil sein kann. Bevorzugt durch Extrudieren herzustellende Formkörper sind Stränge, Hohlstränge oder Hohlstränge mit diametral angeordneten Zwischenwänden.

Bei der Herstellung von Formkörpern durch Granulieren können ebenfalls die üblichen, dem Fachmann hinreichend bekannten Granulierhilfsmittel verwendet werden. Bevorzugt durch Granulieren herzustellende Formkörper sind Kugeln, die im Bereich der Abmessungen von 2 -10 mm liegen. Werden Ringe bzw. Tabletten durch die anderen genannten Verfahren hergestellt, so werden diese Abmessungen von etwa
2 -12 mm für den Außendurchmesser
2 -8 mm für den Innendurchmesser (bei Ringen)
3 -12 mm für die Höhe
aufweisen.

Bei einem anderen Herstellungsweg für .den geformten Katalysator wird zunächst das einzusetzende Titandioxid, welches gegebenenfalls bereits Zusätze von Phosphor, Zirkondioxid, Nioboxid und/oder Antimonoxid enthält, in einem der üblichen genannten Verfahren verformt. Auf diese Formkörper werden anschließend durch ein-oder auch gegebenenfalls mehrmaliges Imprägnieren die Vanadinverbindung durch die Promotoren aufgetränkt. Danach schließt sich eine Trocknung bzw. Calcinierung der getränkten Formkörper an, welche gegebenenfalls auch mehrstufig durchgeführt werden kann, im Bereich von Raumtemperatur bis etwa 550°C in Luft bzw. Stickstoff.

Ein weiterer, bevorzugter Weg zur Herstellung der geformten Katalysatoren besteht darin, die Aktivmasse, bestehend aus feinverteiltem Titandioxid und einer Vanadiumverbindung sowie gegebenenfalls Promotoren, auf einen inerten Träger aufzubringen, in der Weise, daß der Träger nach dem Trocknen in einer Schichtdicke von 0,02 bis 2 mm, vorzugsweise 0,05 bis 0,5 mm, mit der Aktivmasse beschichtet ist.

Das katalytisch aktive Material wird nach üblichen Beschichtungsverfahren auf den Träger aufgebracht.

Dabei wird zum Beispiel der Träger in einer zur Erhitzung von außen eingerichteten Drehtrommel vorgelegt und auf den Träger, während die Temperatur bei etwa 120 bis 350°C gehalten wird, das katalytisch aktive Material, beispielsweise in Form einer Aufschlämmung, aufgesprüht. Eine geeignete Menge des katalytisch aktiven Materials auf dem Träger beträgt etwa 3 bis 30 Gewichtsprozent, in Abhängigkeit von der Größe des Trägers auch weniger bzw. mehr, bezogen auf den fertigen Katalysator. Das katalytisch aktive Material liegt in einer Schichtdicke von 0,02 bis 2 mm, vorzugsweise von 0,05 bis 0,5 mm auf dem inerten Träger vor.

Der nach dem Aufsprühen erhaltene Katalysator wird anschließend bei 250 bis 550°C, vorzugsweise bei 300 bis 500°C, während 0,1 bis 10 Stunden in einem Luftstrom calciniert.

Die zu verwendenden Träger weisen Formen wie Kugeln, Hohlkugeln, Schalen, Säulen oder Ringe auf. Kugeln liegen im Bereich der Abmessungen von 2 -10 mm, während Ringe bzw. Säulen Abmessungen im Bereich von etwa:
4 -12 mm für den Außendurchmesser
2 -8 mm für den Innendurchmesser
3 -12 mm für die Höhe
aufweisen.

Diese Träger können aus inerten, nichtporösen Materialien wie Eisen, Stahl, Aluminium, Porzellan, Tonerde, Aluminiumoxid, Siliziumcarbid oder Silikaten wie Magnesiumsilikat, Aluminiumsilikat oder Zirkonsilikat bestehen. Von Fall zu Fall kann es sich jedoch auch, z.B. zur Steuerung der Aktivität bzw. der Selektivität, von Vorteil erweisen, poröse Materialien bzw. poröse Träger zu verwenden. Solche Materialien können eine scheinbare Porosität bis zu 40 % aufweisen. Als solche Materialien kommen zum Beispiel $\alpha$-Aluminiumoxid oder die sogenannten Übergangsmetalloxide des Aluminiums in Frage, oder auch nichtporöse Materialien wie z.B. Siliziumcarbid oder Magnesiumsilikat, welche zur Porositätserhöhung noch poröse Materialien wie z.B. Aluminiumoxide enthalten. Besonders geeignet hat sich als Trägermaterial Magnesiumsilikat erwiesen.

Gemäß dem neuen Verfahren kann die in Rohren angeordnete Feststoffschüttung außer dem geformten Katalysator noch Inertmaterial enthalten.

Als Inertmaterialien kommen zum einen Formen und Materialien in Frage, wie sie zur Beschichtung mit Katalysatoraktivmasse Verwendung finden. Andererseits können auch andere Materialien, z.B. Graphit in Form von Split, Strängen, Ringen usw. mit gutem Erfolg verwendet werden. Das Verhältnis von Katalysator zu Inertmaterial kann im Reaktor variiert und den Erfordernissen der Reaktion angepaßt werden. Zweckmäßig liegt am Reaktoranfang die höchste Konzentration an Inertmaterial vor und nimmt dann zum Auslaß des Reaktors hin in Stufen oder kontinuierlich ab. Deshalb können im Reaktor auch mehrere Zonen mit unterschiedlichem Verhältnis von Katalysator zu Inertmaterial vorliegen.

In einer spezifischen Ausführungsform des neuen Verfahrens wird bei einstufiger Fahrweise eine Feststoffschüttung verwendet, die in einer ersten Schicht, welche gegebenenfalls in Zonen mit unterschiedlicher Menge an Inertmaterial unterteilt ist, einen Katalysator der ersten Stufe und in einer zweiten Schicht, gegebenenfalls ebenso in Zonen mit unterschiedlicher Menge an Inertmaterial unterteilt, einen Katalysator der zweiten Stufe enthält.

Der Katalysator der ersten Stufe enthält in Form eines Vollkatalysators oder in Form eines Trägerkatalysators eine Aktivmasse, die aus 1 bis 30 Gew.-%, vorzugsweise 2 bis 10 Gew.-% Vanadiumpentoxid und 60 bis 98,5 Gew.-%, vorzugsweise 86 bis 96,5 Gew.-% Titandioxid, überwiegend in der Anatasmodifikation vorliegend, sowie aus 0,5 bis 10 Gew.-%, vorzugsweise 1,5 bis 4 Gew.-% Antimonoxid besteht. Das verwendete Titandioxid weist die bereits aufgeführten physikalischen Eigenschaften auf und kann zusätzlich Phosphor in einem Anteil von 0,01 bis 1 Gew.-% enthalten. Weiterhin enthält die Aktivmasse des Katalysators der ersten Stufe auf 100 Gewichtsteile der Komponenten Vanadiumpentoxid, Titandioxid und Antimonoxid eine oder mehrere Alkalimetallverbindungen von Natrium, Kalium, Rubidium und/oder Caesium mit einem Anteil zwischen 0,01 und 1 Gew.-%, vorzugsweise zwischen 0,05 bis 0,6 Gew.-%. Die Zone, in der der Katalysator der ersten Stufe im Reaktionsrohr vorliegt, nimmt 20 bis 80 % der Gesamthöhe der Feststoffschüttung im Reaktionsrohr vom Einlaß für das Einsatzgas ein.

Der Katalysator der zweiten Stufe weist eine höhere katalytische Aktivität als der Katalysator der ersten Stufe auf. Seine Aktivmasse weist die Zusammensetzung des Katalysators der ersten Stufe auf, enthält jedoch als Promotor zusätzlich Phosphor in einem Anteil bis zu 1,5 Gew.-%, bevorzugt bis 0,8 Gew.-%, bezogen auf die gesamte Aktivmasse. In einer weiteren Ausführungsform wird auf den Zusatz von Alkaliverbindungen beim Katalysator der zweiten Stufe verzichtet, wobei er jedoch Phosphor im oben beschriebenen Anteil enthält. Da die Steuerung der Katalysatoraktivität mit dem in der Aktivmasse enthaltenen Phosphorgehalt möglich ist, kann der Teil der Feststoffschüttung, die den Katalysator der zweiten Stufe enthält, auch in Zonen unterteilt sein, die den Katalysator der zweiten Schicht mit z.B. ansteigendem Phosphorgehalt aufweist. Die Zone, in der der Katalysator der zweiten Stufe im Reaktionsrohr enthalten ist, nimmt 80 % bis 20 % der Gesamthöhe der Feststoffschüttung im Reaktionsrohr vom Gasausgang zum Reaktoreingang hin ein.

In einer weiteren spezifischen Ausführungsform ist das Reaktionssystem in zwei Stufen unterteilt, die bei Bedarf auch auf unterschiedlichem Temperaturniveau temperiert werden können. Im ersten Reaktor ist der Katalysator der ersten Stufe, gegebenenfalls unterteilt in Zonen mit unterschiedlicher, in der Regel in Produktstromrichtung abnehmender Menge an Inertmaterial, enthalten. Der zweite Reaktor enthält den Katalysator der zweiten Stufe, wobei diese Feststoffschüttung ebenfalls in Zonen unterteilt sein kann, die unterschiedliche Mengen an Inertmaterial und/oder wechselnde Anteile an Phosphor in der Aktivmasse enthalten können.

Durch solche bzw. ähnliche Ausführungsformen gelingt es, das gesamte Verfahren über lange Zeit mit gleichbleibender, hoher Leistung zu betreiben. Außerdem werden dadurch die Produktausbeuten verbessert und können durch diese Maßnahmen auf hohem Niveau gehalten werden.

Die nachfolgenden Beispiele sollen das neue Verfahren erläutern:

1. Herstellung der Katalysatoren

a) 600 g Steatitringe mit einem äußeren Durchmesser von 8 mm, einer Länge von 6 mm und einer Wandstärke von 1,5 mm werden in einer Dragiertrommel auf 260°C erhitzt und mit einer Suspension, bestehend aus 390 g Anatas, mit einer inneren Oberfläche von 11 m²/g und einem Gehalt an Phosphor von ca. 0,16 Gew.-%, 10,8 g $Sb_2O_3$, 73,2 g Vanadyloxalat (Vanadiumgehalt entspricht 41 % $V_2O_5$), 500 g Wasser, 100 g Formamid und 1,09 g Rubidiumcarbonat besprüht, bis das Gewicht der aufgetragenen katalytischen Masse 10 % vom Gesamtgewicht des Katalysators ausmacht. Die so aufgebrachte katalytisch aktive Masse besteht aus 90,34 Gew.-% Titandioxid, 7,0 Gew.-% Vanadiumpentoxid, 2,5 Gew.-% Antimonoxid, 0,18 Gew.-% Rubidium und 0,15 Gew.-% Phosphor.

b) Man verfährt wie unter a) beschrieben, wobei man jedoch anstelle von Rubidiumcarbonat 4,87 g Ammoniumhydrogenphosphat zugibt. Im fertigen Katalysator macht das Gewicht der aufgetragenen Masse 10 Gew.-% vom Gesamtgewicht des Katalysators aus. Die katalytisch aktive Schicht besteht aus 90,2 Gew.-% Titandioxid, 7 Gew.-% Vanadiumpentoxid, 2,5 Gew.-% Antimonoxid und 0,45 Gew.-% Phosphor.

c) 5000 g Anatas mit einer inneren Oberfläche von 11 m²/g werden mit 160 g Kartoffelstärke vermischt, mit Wasser angemaischt und im Kneter eine Stunde verdichtet. Die plastische Masse wird zu Ringen mit 6 mm Außendurchmesser, 2 mm Innendurchmesser und einer Länge von 6 mm extrudiert. Die Formkörper werden zunächst 4 Stunden bei 120°C getrocknet und danach 3 Stunden bei 500°C calciniert.

1000 g dieser Formkörper werden dann mit 410 cm³ einer Lösung imprägniert, die 170,7 g Vanadyloxalat und 5,46 g Rubidiumcarbonat enthält. Nach dem Tränken werden die Formkörper wiederum bei 120°C getrocknet und danach 4 Stunden bei 350°C calciniert. Die Formkörper bestehen aus 93,18 Gew.-% Titandioxid, 6,52 Gew.-% Vanadiumpentoxid und 0,3 Gew.-% Rubidium.

d) 1000 g der nach c) hergestellten Titandioxid-Formkörper werden mit 410 cm³ einer Lösung imprägniert, die 170,7 g Vanadyloxalat und 27,21 g $NH_4H_2PO_4$ enthält. Die imprägnierten Formkörper werden wie unter c) getrocknet und calciniert. Sie bestehen aus 92,8 Gew.-% Titandioxid, 6,5 Gew.-% Vanadiumpentoxid und 0,7 Gew.-% Phosphor.

2. Durchführung der Oxidation

a) 100 Volumenteile der gemäß Beispiel 1a) und 100 Volumenteile des nach Beispiel 1b) hergestellten Katalysators werden in einen Röhrenreaktor mit 25 mm innerem Durchmesser eingefüllt, und so mit einem Einsatzstoffgemisch beaufschlagt, daß zuerst die Katalysatorschicht nach Beispiel 1a) und dann die Katalysatorschicht nach Beispiel 1b) durchströmt wird. Dem Reaktor, dessen Wand auf 340°C beheizt ist, wird auf diese Weise stündlich ein Gemisch aus 540 000 Volumenteilen Luft und 16,2 Gewichtsteilen 4-tertiär-Butyl-o-Xylol zugeführt (Volumenteile verhalten sich zu Gewichtsteilen wie Liter zu Kilogramm). Das den Reaktor verlassende gasförmige Reaktionsgemisch wird auf 30°C abgekühlt, wobei das Reaktionsprodukt und gegebenenfalls nicht umgesetztes Ausgangsprodukt abgeschieden werden. Das Restgas wird mit Wasser gewaschen und nach Abdampfen des Waschwassers der verbleibende Rückstand mit dem abgeschiedenen Produkt vereinigt.

Die Analyse des Produktes ergibt, daß der Ausgangsstoff 4-tertiär-Butyl-o-Xylol vollständig umgesetzt wurde. Bezogen auf 100 g eingesetztes 4-tertiär-Butyl-o-Xylol werden 95,7 g Rohprodukt mit einem Gehalt an 94,8 Gew.-% 4-tertiär-Butyl-Phthalsäureanhydrid, entsprechend 72,0 % der Theorie, gefunden.

b) 100 Volumenteile des nach Beispiel 1c) und 100 Volumenteile des nach Beispiel 1d) hergestellten Katalysators werden in einen Röhrenreaktor mit 25 mm innerem Durchmesser eingefüllt und so mit dem Einsatzstoffgemisch beaufschlagt, daß zuerst die Katalysatorschicht nach Beispiel 1c) und dann die Katalysatorschicht nach Beispiel 1d) durchströmt wird. Dem Reaktor, dessen Wand auf 325°C beheizt ist, wird auf diese Weise stündlich ein Gemisch aus 450 000 Volumenteilen Luft und 11,2 Gewichtsteilen 4-tertiär-Butyl-o-Xylol zugeführt. Das den Reaktor verlassende Reaktionsgemisch wird entsprechend Beispiel 2a) aufgearbeitet.

Die Analyse des Produktes ergibt, daß der Ausgangsstoff vollständig umgesetzt wurde. Bezogen auf 100 g Einsatzprodukt werden 85,5 g Rohprodukt mit einem Gehalt an 92,3 Gew.-% 4-tertiär-Butyl-Phthalsäureanhydrid, entsprechend 65,6 % der Theorie, gefunden.

c) 100 Volumenteile des nach Beispiel 1a) hergestellten Katalysators werden in einen Röhrenreaktor mit 25 mm innerem Durchmesser eingefüllt, dessen Wand auf 343°C beheizt ist. Der Reaktor wird stündlich mit einem Gemisch aus 600 000 Volumenteilen Luft und 28 Gewichtsteilen 4-tertiär-Butyl-o-Xylol beaufschlagt. Das den Reaktor verlassende Reaktionsgemisch wird einem zweiten Reaktor mit gleichen Dimensionen zugeführt, der 100 Volumenteile des nach Beispiel 1b) hergestellten Katalysators enthält und dessen Wand auf 300°C beheizt ist. Das diesen Reaktor verlassende Reaktionsgemisch wird entsprechend Beispiel 2a) aufgearbeitet.

Die Analyse des Produktes ergibt, daß der Einsatzstoff vollständig umgesetzt wurde. Bezogen auf 100 g Einsatzprodukt werden 96,3 g Rohprodukt mit einem Gehalt an 95,9 Gew.-% 4-tertiär-Butyl-Phthalsäureanhydrid, entsprechend 73,3 % der Theorie, gefunden.

**Ansprüche**

1. Verfahren zur Herstellung von 4-tertiär-Butyl-Phthalsäureanhydrid durch Oxidation von 4-tertiär-Butyl-o-Xylol mit Luft, Sauerstoff oder Sauerstoff enthaltenden Gasen in Gegenwart von Katalysatoren, dadurch gekennzeichnet, daß ein geformter Katalysator verwendet wird, der als aktive Komponenten Titandioxid, überwiegend in der Anatasmodifikation und Vanadiumpentoxid enthält.

2. Verfahren nach Anspruch 1, dadurch gekennzeichnet, daß man Katalysatoren verwendet, die zusätzlich als Promotoren Antimon, Niob, Alkalimetalle, Phosphor und Zirkon oder Verbindungen dieser Elemente enthalten.

3. Verfahren nach Anspruch 1 und 2, dadurch gekennzeichnet, daß man Katalysatoren verwendet, die die Aktivmasse als Schale auf einem inerten Träger aus Magnesiumsilikat, vorzugsweise in Ringform oder Schalenform, aufgetragen enthält.

4. Verfahren nach Anspruch 1 bis 3, dadurch gekennzeichnet, daß der geformte Katalysator 1 bis 30 Gew.-%, vorzugsweise 2 bis 10 Gew.-% Vana-

diumpentoxid, 70 bis 99 Gew.-%, vorzugsweise 90 bis 98 Gew.-% Titandioxid, sowie bezogen auf die Gesamtmasse an Vanadiumpentoxid und Titandioxid 0,01 bis 1 Gew.-%, vorzugsweise 0,05 bis 0,6 Gew.-% eines oder mehrerer Alkalimetalle, vorzugsweise Rubidium und/oder bis zu 1,5 Gew.-%, vorzugsweise bis zu 0,8 Gew.-% Phosphor enthält.

5. Verfahren nach Anspruch 1 bis 3, dadurch gekennzeichnet, daß der geformte Katalysator 1 bis 30 Gew.-%, vorzugsweise 2 bis 10 Gew.-% Vanadiumpentoxid, 60 bis 98,5 Gew.-%, vorzugsweise 86 bis 96,5 Gew.-% Titandioxid, 0,5 bis 10 Gew.-%, vorzugsweise 1,5 bis 4 Gew.-% Antimonoxid sowie bezogen auf die Gesamtmasse aus Vanadiumpentoxid, Titandioxid und Antimonoxid aus 0,01 bis 1 Gew.-%, vorzugsweise 0,05 bis 0,6 Gew.-% eines oder mehrerer Alkalimetalle, vorzugsweise Rubidium und/oder aus bis zu 1,5 Gew.-%, vorzugsweise bis zu 0,8 Gew.-% Phosphor enthält.

6. Verfahren nach Anspruch 1 bis 5, dadurch gekennzeichnet, daß das Titandioxid überwiegend in der Anatasmodifikation mit einer spezifischen Oberfläche nach BET von 7 bis 100 m²/g, vorzugsweise von 9 bis 60 m²/g vorliegt.

7. Verfahren nach Anspruch 1 bis 6, dadurch gekennzeichnet, daß das Titandioxid Phosphor in einem Anteil von 0,01 bis 0,3 Gew.-%, vorzugsweise bis 0,2 Gew.-%, enthält.

8. Verfahren nach Anspruch 1 bis 7, dadurch gekennzeichnet, daß der Katalysator Ringform mit einem Außendurchmesser von 4 bis 20 mm, eine Höhe von 3 bis 20 mm und eine Wandstärke von mindestens 1 mm hat.

9. Verfahren nach Anspruch 1 bis 7, dadurch gekennzeichnet, daß der Katalysator in Rohren mit einem inneren Durchmesser von 15 bis 40 mm, vorzugsweise von 19 bis 30 mm angeordnet ist.

10. Verfahren nach Anspruch 1 bis 9, dadurch gekennzeichnet, daß die Katalysatorschüttung unterteilt ist in mindestens zwei Zonen, wobei in Gasströmungsrichtung in der ersten Zone, die 20 bis 80 % der Gesamthöhe im Reaktorrohr ausmacht, ein geformter Katalysator verwendet wird, der 1 bis 30 Gew.-%, vorzugsweise 2 bis 10 Gew.-% Vanadiumpentoxid, 60 bis 98,5 Gew.-%, vorzugsweise 86 bis 96,5 Gew.-% Titandioxid, 0,5 bis 10 Gew.-%, vorzugsweise 1,5 bis 4 Gew.-% Antimonoxid sowie bezogen auf die Gesamtmasse aus Vanadiumpentoxid, Titandioxid und Antimonoxid 0,01 bis 1 Gew.-%, vorzugsweise 0,05 bis 0,6 Gew.-% eines oder mehrere Alkalimetalle, vorzugsweise Rubidium enthält sowie in einer zweiten Zone, die den Rest des Reaktionsrohres ausfüllt, ein geformter Katalysator verwendet wird, der 1 bis 30 Gew.-%, vorzugsweise 2 bis 10 Gew.-% Vanadiumpentoxid, 60 bis 98,5 Gew.-%, vorzugsweise 86 bis 96,5 Gew.-% Titandioxid, 0,5 bis 10 Gew.-

%, vorzugsweise 1,5 bis 4 Gew.-% Antimonoxid sowie bezogen auf die Gesamtmasse aus Vanadiumpentoxid, Titandioxid und Antimonoxid Phosphor einen Anteil bis zu 1,5 Gew.-%, vorzugsweise bis zu 0,8 Gew.-% enthält.

11. Verfahren nach Anspruch 1 bis 9, dadurch gekennzeichnet, daß die Katalysatorschüttung unterteilt ist in mindestens zwei Zonen, wobei in Gasströmungsrichtung in der ersten Zone, die 20 bis 80 % der Gesamthöhe im Reaktionsrohr ausmacht ein Katalysator verwendet wird, der auf einem inerten, vorzugsweise ringförmigen Träger, eine Schale einer Aktivmasse enthält, die aus 1 bis 30 Gew.-%, vorzugsweise 2 bis 10 Gew.-% Vanadiumpentoxid, 60 bis 98,5 Gew.-%, vorzugsweise 86 bis 96,5 Gew.-% Titandioxid, 0,5 bis 10 Gew.-%, vorzugsweise 1,5 bis 4 Gew.-% Antimonoxid sowie bezogen auf die Gesamtmasse aus Vanadiumpentoxid, Titandioxid und Antimonoxid 0,01 bis 1 Gew.-%, vorzugsweise 0,05 bis 0,6 Gew.-% eines oder mehrerer Alkalimetalle, vorzugsweise Rubidium besteht sowie in einer zweiten Zone, die den Rest des Reaktionsrohres ausfüllt, ein Katalysator verwendet wird, der auf einem inerten, vorzugsweise ringförmigen Träger eine Schale einer Aktivmasse enthält, die aus 1 bis 30 Gew.-%, vorzugsweise 2 bis 10 Gew.-% Vanadiumpentoxid, 60 bis 98,5 Gew.-%, vorzugsweise 86 bis 96,5 Gew.-% Titandioxid, 0,5 bis 10 Gew.-%, vorzugsweise 1,5 bis 4 Gew.-% Antimonoxid sowie bezogen auf die Gesamtmasse aus Vanadiumpentoxid, Titandioxid und Antimonoxid Phosphor in einem Anteil bis zu 1,5 Gew.-%, vorzugsweise bis zu 0,8 Gew.-% besteht.

12. Verfahren nach Anspruch 1 bis 11, dadurch gekennzeichnet, daß der gegebenenfalls in Zonen angeordnete Katalysator mit Inertmaterial verdünnt wird.

13. Verfahren nach Anspruch 1 bis 12, dadurch gekennzeichnet, daß die Reaktionszone in zwei Zonen bzw. 2 Reaktoren unterteilt ist, die auf unterschiedlichem Temperaturniveau gehalten werden in der Weise, daß man die zweite Zone bzw. den zweiten Reaktor auf niedrigerer Temperatur als die erste Zone bzw. den ersten Reaktor hält.

Europäisches
Patentamt

# EUROPÄISCHER RECHERCHENBERICHT

Nummer der Anmeldung

EP 86 11 6989

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-4 046 780 (NAKANISHI et al.) <br> * Ansprüche 1-9; Spalte 2, Zeile 60 - Spalte 3, Zeile 5; Spalte 4, Zeilen 3-11; Spalte 5, Zeilen 62-68 * | 1-13 | C 07 D 307/89 |
| | --- | | |
| Y | DE-A-1 769 998 (B.A.S.F.) <br> * Ansprüche 1,3-5; Seite 3, Zeile 1 - Seite 4, Zeile 12 * | 1-5,8 | |
| | --- | | |
| Y | DE-A-1 792 123 (B.A.S.F.) <br> * Anspruch; Seiten 3,4, Beispiel * | 1-3 | |
| | --- | | |
| Y | US-A-3 909 457 (FRIEDRICHSEN et al.) <br> * Ansprüche 1-4; Spalte 1, Zeile 61 - Spalte 2, Zeile 66 * | 1-5,8 9 | **RECHERCHIERTE SACHGEBIETE (Int. Cl.4)** |
| | --- | | C 07 C 51/00 <br> C 07 C 63/00 |
| Y | DE-A-3 045 624 (NIPPON SHOKUBAI) <br> * Ansprüche 1-5; Seite 10, Zeilen 7-11,23-31; Seite 11, Zeilen 1-5, Zeile 12 - Seite 12, Zeile 12 * | 1-13 | |
| | ---     -/- | | |

Der vorliegende Recherchenbericht wurde für alle Patentansprüche erstellt.

| Recherchenort <br> DEN HAAG | Abschlußdatum der Recherche <br> 20-03-1987 | Prüfer <br> KLAG M.J. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 93, Nr. 5, 4. August 1980, Seite 879, Zusammenfassung Nr. 46149d, Columbus, Ohio, US; A.A. SHAPOVALOV et al.: "Preparation of 4-tert-butylphthalic anhydride by the vapor-phase catalytic oxidation of tert-butyl derivatives of o-xylene and naphthalene", & ZH. PRIKL. KHIM.(LENINGRAD) 1980, 53(2), 418-20 ----- | 1 | |

RECHERCHIERTE
SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| DEN HAAG | 20-03-1987 | KLAG M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503 03 82